# FASCICULE DE BREVET EUROPEEN

(11) **EP 2 659 921 B1**
(45) Date de publication et mention de la délivrance du brevet: **14.06.2017**
(21) Numéro de dépôt: 13165850.2
(22) Date de dépôt: 29.04.2013
(51) Int. Cl.: A61M 5/00, A61M 5/142, G09F 3/02, A61J 1/05, A61J 1/10, G06F 19/00, A61M 5/14, A61M 5/31

(54) **Kit de recharge d'une pompe a perfusion comprenant réservoir de médicament et étiquette détachable**
Wiederbefüllset für eine Infusionspumpe mit Medikamentenreservoir und ablösbarem Etikett
Kit for recharging an infusion pump with drug reservoir and detachable label

(30) Priorité: 02.05.2012 FR 1254018
(43) Date de publication de la demande: 06.11.2013
(73) Titulaire: Pharmadyne, 75009 Paris (FR)
(72) Inventeur: Rosoor, Lucas, 94130 Nogent Sur Marne (FR); Malfait, Benjamin, 75013 Paris (FR)
(74) Mandataire: Cabinet Plasseraud

(56) Documents cités:
- EP-A1- 1 433 456
- WO-A1-2005/032449
- WO-A1-2005/089835
- WO-A2-2010/078084
- US-A- 4 795 429
- US-A1- 2002 143 320
- US-A1- 2005 101 905
- US-A1- 2005 119 622

## Description

La présente invention concerne un kit de recharge d'une pompe à perfusion comprenant un conteneur prêt à l'emploi contenant un médicament à administrer par perfusion, notamment un analgésique de niveau III. Ce kit procure une sécurité accrue lors de la recharge d'une pompe à perfusion. La présente invention concerne également une méthode sécurisée d'utilisation du kit de recharge et une méthode sécurisée de préparation du kit de recharge. Un autre objet de la présente invention porte sur un dispositif sécurisé de délivrance d'un médicament par perfusion.

L'antalgie autocontrôlée (PCA : Patient Controlled Analgesia, ou ACP : Antalgie Contrôlée par le Patient) est une technique qui permet au malade de s'auto-administrer à l'aide d'une pompe programmable des doses prédéterminées d'antalgique par voie parentérale (intraveineuse ou sous-cutanée). Dans la présente demande les termes analgésique et antalgique sont utilisés indifféremment pour définir un antidouleur. La PCA, mise au point initialement pour la douleur aiguë postopératoire en vue de l'administration exclusive de bolus, est fréquemment utilisée en douleur cancéreuse, où elle associe débit de base ou perfusion continue, et bolus ou interdose. Par « débit de base » on entend le débit de solution médicamenteuse perfusée en continu. Il est généralement de 0,3 à 2 ml/h pour la morphine. Par « bolus » ou « interdose » on entend une injection rapide d'une quantité prédéterminée de solution médicamenteuse. C'est la dose que reçoit un patient quand il appuie sur le bouton poussoir.

Les conditions d'administration se font selon une programmation prédéterminée par le médecin prescripteur grâce à un système informatisé inclus dans une pompe à perfusion portable. La pompe est verrouillable pour éviter que le malade ou son entourage ne modifie la programmation. Le principe actif est contenu dans un réservoir comme une cassette ou une poche souple jetable d'une capacité de 50 à 250 ml. La concentration du principe actif à administrer est adaptée à la posologie souhaitée en fonction des paramètres de la pompe. Dans le cas d'analgésiques de type III notamment, le principe actif disponible sous forme d'ampoules est préparé à la concentration souhaitée par mélange avec une solution pour perfusion directement dans un réservoir avant mise en place du système de perfusion. Ces opérations manuelles sont contraignantes et doivent généralement être réalisées par du personnel médical.

Malgré les systèmes de sécurité mis en place sur les pompes à perfusion. Le nombre d'opérations manuelles lors de la mise en place ou du renouvellement d'un système de perfusion reste important et peut être source d'erreurs par exemple lors de la préparation de la solution médicamenteuse à perfuser (utilisation d'une ampoule de médicament dont la capacité et/ou la concentration ne correspond pas à la prescription, ou d'un réservoir dont la capacité ne correspond pas à la prescription). Ces opérations augmentent les risques d'erreur sur la concentration finale de la solution médicamenteuse à perfuser. Des erreurs peuvent survenir également lors de la programmation de la pompe par le personnel médical. Dans le cas notamment d'administration d'analgésiques de classe III, tels que la morphine, ces erreurs peuvent provoquer des accidents de surdosage et avoir de graves conséquences sur le patient.
D1 (US 2005/119622) décrit une unité de stockage pour organiser et stocker des doses prédéterminées de médicament.
D2 (US 4 795 429) décrit une méthode et un appareil pour augmenter la précision de l'administration intraveineuse d'une pluralité de flux médicinaux.
D3 (US 2005/101905) décrit une méthode et un appareil pour coder des seringues, fioles ou ampoules.
D4 (US 2002/143320) décrit un système et un procédé de suivi de produits médicaux via une identification de fréquence radio.
D5 (EP 1 433 456) un dispositif d'injection dans lequel le temps et le débit d'une administration peuvent être enregistrés.
D6 (WO 2005/089835) décrit une pompe à seringue munie d'un lecteur radiofréquence permettant de lire un marqueur de radiofréquence codé sur la seringue.
D7 (WO 2010/078084) décrit un système pour identifier un réservoir utilisé dans un dispositif de distribution d'un fluide.
D8 (WO 2005/032449) décrit un récipient pour un dispositif d'administration de médicament comprenant un lecteur optique.

Il est donc souhaitable pour améliorer la sécurité du patient de réduire le nombre d'opérations manuelles lors de la mise en place d'un système PCA mais également de développer des systèmes permettant de détecter les éventuelles erreurs pouvant survenir lors de la mise en place d'un système de perfusion.

Dans ce contexte, la Demanderesse a mis au point un kit de recharge d'une pompe à perfusion répondant à ces attentes.
FIG. 1 est une représentation, en vue de dessus, d'un mode de réalisation du kit de recharge d'une pompe à perfusion selon l'invention dans, un arraché permettant de voir les composants du kit à l'intérieur de l'emballage.
FIG. 2 est une représentation d'un réservoir sur lequel est positionnée l'étiquette détachable du kit de la FIG. 1.
FIG. 3 représente une pompe à perfusion dans laquelle a été placée, avec la méthode selon l'invention, le réservoir de la FIG. 2.

Le kit de recharge d'une pompe à perfusion selon l'invention est décrit par la revendication indépendante 1 et, entre autre, il comprend :
- un emballage comprenant une étiquette détachable ; et
- un réservoir contenant une solution médicamenteuse à administrer par perfusion ;
caractérisé en ce qu'un emplacement est prévu à la surface du réservoir, ledit emplacement étant adapté pour recevoir l'étiquette détachable, et ladite étiquette détachable comprend des moyens d'identification autres qu'un message écrit.

Le réservoir peut être notamment une cassette ou une poche souple. Le réservoir comprend au moins un orifice permettant l'arrivée ou la sortie de la solution médicamenteuse qu'il est amené à contenir. Le réservoir est réalisé en matériau pharmaceutiquement acceptable. Par « matériau pharmaceutiquement acceptable » on entend tout matériau n'ayant pas d'effet indésirable sur la solution à perfuser avec laquelle il est en contact et n'entraîne aucune manifestation de toxicité générale ni de réaction locale sur la personne recevant la solution à perfuser.

De préférence, le réservoir est une poche souple. La poche souple peut être constituée d'une membrane multicouche en matériau souple formant un volume intérieur. La membrane multicouche peut être constituée d'une gaine soudée, d'une feuille pliée et soudée ou d'un assemblage de plusieurs feuilles soudées entre elles, de préférence deux feuilles, de manière à délimiter un volume intérieur. La membrane multicouche comprend au moins une couche réalisée dans un matériau empêchant toute migration de composés de l'intérieur de la poche vers l'extérieur ou inversement. La membrane comprend notamment une couche imperméable à l'oxygène. Des matériaux adaptés à la couche imperméable à l'oxygène sont, par exemple, les polyoléfines (polyéthylène, polypropylène), le poly(chlorure de vinyle) (PVC) et poly(éthylène-acétate de vinyle) (EVA). La poche présente généralement une surface interne lisse mais peut également présenter une surface texturée. L'avantage d'une surface interne texturée est que, lorsque la quantité de solution médicamenteuse diminue, les deux parois de la poche ne se collent pas et sont moins susceptibles d'empêcher l'écoulement de la solution vers l'orifice. Une telle surface interne peut présenter par exemple une surface striée ou granulée obtenue notamment par extrusion ou par moulage. La membrane multicouche est de préférence transparente. Cela permet d'examiner visuellement à tout moment l'état de la solution contenue dans la poche. L'orifice est de préférence doté d'un raccord et d'une valve anti retour.

Le réservoir du kit selon l'invention comprend un emplacement adapté pour recevoir l'étiquette détachable. Par « emplacement adapté pour recevoir l'étiquette détachable » on entend un emplacement prédéterminé ayant notamment la forme et la taille de l'étiquette détachable. Cet emplacement peut être signalé sur la poche notamment par une marque définissant un contour ayant la forme et la taille de l'étiquette détachable, par une couleur différente du reste du réservoir et/ou par une texture différente du reste de la surface externe du réservoir. Cet emplacement doit être recouvert par l'étiquette détachable avant la mise en place du système de perfusion. De préférence, une alerte visuelle (symbole ou message de danger) est présente sur l'emplacement de façon à ce que l'utilisateur (personnel médical ou patient) soit obligé de coller l'étiquette détachable pour faire disparaître cette alerte. Cet emplacement est de préférence situé sur une partie de la poche ne gênant pas l'examen visuel du contenu de la poche.

Le réservoir contient une solution médicamenteuse à administrer par perfusion. En d'autres termes, le réservoir contient une solution médicamenteuse prête à l'emploi : il ne nécessite par conséquent pas d'opérations de dilution supplémentaire lors de la mise en place ou du renouvellement de la perfusion, ce qui permet de s'affranchir des risques d'erreur lors de ces opérations.

La solution médicamenteuse à administrer par perfusion est une solution physiologique contenant une concentration prédéterminée de principe actif. La solution physiologique est par exemple une solution de NaCl diluée à 9 pour 1000 dans de l'eau distillée. Le principe actif peut être tout principe actif administrable par perfusion, de préférence un antalgique, plus préférentiellement un antalgique de niveau III tel que défini dans la classification OMS des antalgiques, notamment la morphine, l'hydromorphine, l'oxycodone, le fentanyl, la buprénorphine, la nalbuphine et la pentazocine.

L'emballage comprend le réservoir et éventuellement d'autres composants. Le réservoir, et éventuellement d'autres composants du kit, peuvent être contenus dans des emballages individuels, de préférence stériles.

L'étiquette détachable est attachée à la surface de l'emballage, de préférence à la surface externe de l'emballage principal du kit. L'étiquette détachable peut être solidaire de l'étiquette règlementaire contenant les renseignements écrits concernant le produit mais elle sera de préférence dissociable de l'étiquette règlementaire. De préférence, elle est autocollante. Une fois détachée, elle présente de préférence une face autoadhésive permettant de la maintenir en place une fois repositionnée sur l'emplacement du réservoir adapté pour la recevoir. L'étiquette détachable comprend des moyens d'identification autre qu'un message écrit. Par l'expression « moyen d'identification » au sens de la présente invention on entend tout moyen autre qu'un message écrit permettant d'identifier un produit par des informations qui y sont associées. Les moyens d'identification peuvent être un symbole imprimé sur l'étiquette (code couleur, code barre, code en deux dimensions,...) auquel des informations sont associées via l'établissement d'un code, ou un support de stockage solidaire de l'étiquette et permettant le stockage de données informatiques. Avantageusement, les moyens d'identification sont constitués d'une puce RFID (Radio Frequency IDentification). Ces moyens d'identification sont associés notamment à des informations (IE) sur la solution médicamenteuse à perfuser, telles que la nature du principe actif, la concentration de celui-ci, la capacité du réservoir, la date limite d'utilisation ou le numéro de lot. Ce type d'information peut être enregistré dans les moyens d'identification au moment de la préparation du kit. D'autres informations peuvent être ajoutées ultérieurement par exemple lors de la vente du kit.

S'agissant des analgésiques de classe III, ces substances présentent un caractère stupéfiant et la sécurisation de la délivrance et de la traçabilité de ces substances est un enjeu important. Par conséquent, pour permettre un contrôle et une traçabilité de ces substances, la réglementation impose que les informations sur le nom du patient et la référence de la prescription soient reportées sur tout produit les contenant lors de son utilisation. En d'autres termes, dans les dispositifs classiques, ces mentions doivent apparaître sur l'emballage externe contenant l'ampoule de principe actif, puis, en théorie, reportées sur l'ampoule quand l'emballage est ouvert, puis sur le réservoir dans lequel la dilution est effectuée. Cependant, un oubli rend le traçage de ces substances impossible. Selon l'invention, l'étiquette détachable comprend de préférence un emplacement destiné à l'inscription du nom du patient et la référence de la prescription. Ces informations peuvent également être ajoutées par exemple lors de la vente du kit dans les moyens d'identification de l'étiquette détachable. Ces informations sont transférées par décollage et recollage de l'étiquette lors de l'utilisation.

Le kit selon l'invention peut comprendre une tubulure. Cette tubulure permet de relier le réservoir et la pompe. Elle doit donc être adaptée au type de pompe à perfusion utilisé. Elle est faite d'un matériau souple pharmaceutiquement acceptable, tel que le PVC, le polyéthylène, le polyuréthane, le silicone ou le polypropylène. La tubulure peut comprendre des valves anti retour et des raccords à chacune de ses extrémités. Elle peut également comprendre un élément détrompeur adapté au type de pompe auquel elle est destinée.

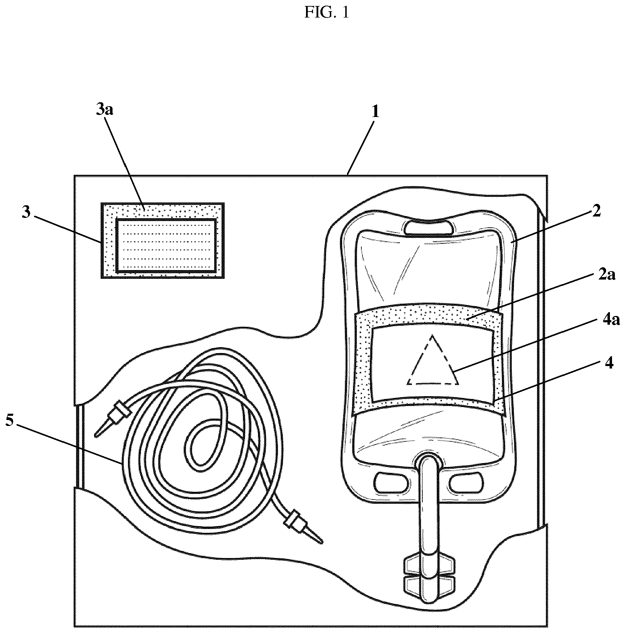

Dans un mode de réalisation particulier représenté en FIG. 1, le kit de recharge d'une pompe à perfusion selon l'invention comprend :
- un emballage **1** comprenant un réservoir **2** contenant une solution médicamenteuse à administrer par perfusion ; et
- une étiquette détachable **3** attachée à l'emballage **1 ;**
caractérisé en ce qu'un emplacement **4** est prévu à la surface du réservoir, ledit emplacement **4** étant prédéterminé pour recevoir l'étiquette détachable **3 ;**
ladite étiquette détachable **3** comprend des moyens d'identification visuels (IVE) **3a** autres qu'un message écrit ; et
ledit réservoir comprend des moyens d'identification visuels (IVR) **2a** autres qu'un message écrit.

L'emplacement **4** peut comprendre une alerte visuelle **4a**.

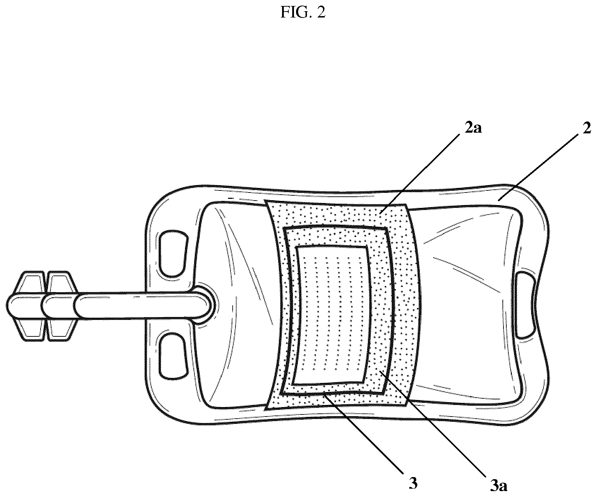

Dans ce mode de réalisation particulier, les moyens d'identification de l'étiquette détachable **3** comprennent ou sont constitués par des moyens d'identification visuels (IVE) **3a** associés aux informations (IE) relatives à la solution médicamenteuse à perfuser annoncées sur l'emballage, notamment la nature du principe actif et/ou à la concentration de celui-ci. Ils sont disposés sur au moins une partie de l'étiquette **3,** par exemple sur le pourtour. Ces moyens d'identification visuels peuvent être des symboles prédéterminés, un code couleur prédéterminé ou une combinaison de ceux-ci.. Le réservoir **2** comprend également des moyens d'identification visuels (IVR) **2a** répondant au même code prédéterminé que les moyens d'identification visuels de l'étiquette (IVE) **3a.** Les moyens d'identification visuels (IVR) 3a sont associés à des informations (IR) relatives à la solution médicamenteuse contenue dans le réservoir **2.** De préférence, les moyens d'identification visuels du réservoir (IVR) **2a** sont disposés au voisinage de l'emplacement **4** prévu pour recevoir l'étiquette détachable **3,** de façon à ce que lesdits moyens d'identification visuels (IVR) **2a** restent visibles au moins en partie lorsque l'étiquette **3** est positionnée sur l'emplacement **4,** comme illustré par FIG. 2.

De manière avantageuse, les autres éléments éventuellement présents dans le kit peuvent également comprendre des moyens d'identification visuels répondant au même code prédéterminé que les moyens d'identification visuels de l'étiquette (IVE). Notamment, la tubulure **5** peut comprendre des moyens d'identification visuels (IVT) (non représentés sur la FIG. 1) répondant au même code prédéterminé que les moyens d'identification visuels de l'étiquette (IVE) **3a.**

Les moyens d'identification visuels sont de préférence un code couleur prédéterminé. Dans ce mode de réalisation préféré, une échelle de couleur peut être prédéterminée de façon à associer une couleur donnée à une concentration de principe actif donnée. L'étiquette détachable est au moins en partie colorée, par exemple sur son pourtour, de la couleur (IVE) correspondant à la concentration de la solution médicamenteuse à perfuser annoncée sur l'emballage du kit (IE). Le réservoir est au moins en partie coloré de la couleur (IVR) correspondant à la concentration de la solution médicamenteuse à perfuser qu'il contient (IR). De préférence, le réservoir est au moins coloré au voisinage de l'emplacement prédéterminé pour recevoir l'étiquette, par exemple sur son pourtour. Lorsque l'utilisateur positionne l'étiquette détachable sur l'emplacement du réservoir, il compare nécessairement la couleur (IVE) de l'étiquette et la couleur (IVR) du réservoir et peut vérifier directement et simplement si la concentration de la solution médicamenteuse contenue dans le réservoir (IR) correspond à la concentration annoncée sur l'emballage du kit (IE). Tel sera le cas si les couleurs sur l'étiquette (IVE) et sur le réservoir (IVR) sont identiques.

Le code couleur mentionné ci-dessus peut être remplacé ou combiné de manière équivalente par d'autres moyens d'identifications visuels tels que des symboles, un symbole donné étant associé à une concentration de principe actif donné. D'autres moyens d'identification visuels peuvent également être combinés au code couleur en étant associé à des informations différentes. Par exemple des symboles peuvent être combinés au code couleur pour identifier la nature du principe actif, un symbole donné étant alors associé à un principe actif donné.

Ainsi, le kit selon l'invention permet à un utilisateur n'ayant aucune formation spécifique de distinguer directement et simplement si les informations annoncées sur l'emballage (IE) sont en adéquation avec les informations de la solution médicamenteuse effectivement contenue dans le réservoir (IR). Dans le cas où les moyens d'identification visuels de l'étiquette (IVE) et du réservoir (IVR) correspondent, l'utilisateur peut se fier aux informations (IE) annoncées sur l'emballage qui correspondent effectivement aux informations (IR) relatives à la solution médicamenteuse contenue dans le réservoir. Du fait de la simplicité de la comparaison des informations de l'emballage (IE) et du réservoir (IR) avec le kit selon l'invention, l'utilisateur, même non averti, vérifie pratiquement inévitablement et de manière systématique leur correspondance. Ainsi, cette vérification fait office de détrompeur. Le kit selon l'invention permet donc de prévenir les risques liés aux erreurs de conditionnement.

La présente invention concerne également une méthode sécurisée d'utilisation du kit de recharge d'une pompe à perfusion comprenant les étapes suivantes :
- détacher de l'emballage l'étiquette détachable comprenant des moyens d'identification et positionner ladite étiquette détachable sur le réservoir à l'emplacement adapté pour recevoir l'étiquette détachable ; lesdits moyens d'identification contenant des informations (IE) relatives à la solution médicamenteuse à administrer par perfusion ;
- positionner le réservoir dans une pompe à perfusion programmable ;
- programmer la pompe à perfusion programmable avec des informations (IP) relatives à la solution médicamenteuse à administrer par perfusion ;
- détecter les informations (IE) contenues dans les moyens d'identification ; et
- comparer les informations détectées (IE) et les informations préprogrammées (IP).

La méthode sécurisée d'utilisation du kit de recharge d'une pompe à perfusion selon l'invention n'est pas une méthode de traitement. Notamment, la méthode selon l'invention exclue les étapes de mise en place de la perfusion sur un patient et d'administration de la solution médicamenteuse au patient.

L'étiquette détachable doit être détachée de l'emballage et positionnée sur l'emplacement du réservoir adapté pour recevoir celle-ci.

De préférence, une alerte visuelle (symbole de danger ou message de danger) est présente sur le réservoir à l'emplacement adapté pour recevoir l'étiquette détachable, et l'étiquette détachable est positionnée sur le réservoir audit emplacement de façon à faire disparaître l'alerte visuelle présente à cet emplacement. Cette alerte visuelle a pour avantage d'inciter l'utilisateur à positionner l'étiquette détachable sur le réservoir. Ce faisant, l'utilisateur voit si les informations concernant le nom du patient et la référence de la prescription sont inscrites sur l'étiquette et est incité à les inscrire si elles sont absentes. La présence de l'alerte visuelle diminue ainsi les risques d'oubli et améliore la sécurité d'utilisation du kit et l'efficacité de la traçabilité.

L'étiquette détachable comprend des moyens d'identification tels que définis ci-dessus. Ces moyens d'identification contiennent des informations (IE) notamment relatives à la solution médicamenteuse à perfuser (nature du principe actif, concentration de celui-ci, capacité du réservoir, date limite d'utilisation, numéro de lot...). Lorsque les moyens d'identification sont un support de stockage de données, des informations relatives aux conditions d'administration (débit de base, dose d'un bolus, le temps minimal entre deux bolus, le nombre de bolus par heure ou la dose limite de principe actif autorisée par heure,...) telles que décrites sur la prescription peuvent également être ajoutées aux moyens d'identification lors de la mise en place du système de perfusion.

La pompe à perfusion programmable peut être n'importe quelle pompe à perfusion programmable permettant la mise en place d'un système de perfusion, de préférence d'un système de perfusion ambulatoire. De telles pompes à perfusion disponibles dans le commerce sont, par exemple, les pompes Rythmic™ commercialisées par la société Micrel, SYNCHROMED® commercialisées par la société Medtronic, GemStar® commercialisées par la société Hospira ou BodyGuard commercialisée par la société Ceasarea Medical Electronics (CME) Cadd commercialisée par la société Smith Medical.

La pompe est généralement programmée par le personnel médical et est de préférence verrouillée pour éviter que le patient ou son entourage ne puisse modifier la programmation. Les informations (IP) relatives à la solution médicamenteuse à perfuser comprennent, par exemple, des informations sur la nature du principe actif à administrer, la concentration du principe actif dans la solution médicamenteuse à perfuser ou la capacité du réservoir contenant la solution médicamenteuse à perfuser. Les informations (IP) préprogrammées contiennent également des informations relatives aux conditions d'administration du principe actif, telles que le débit de base de la perfusion, la quantité de perfusion pour les bolus, le temps minimal entre deux bolus, le nombre de bolus par heure ou la dose limite de principe actif autorisée par heure. Elles peuvent également comprendre des informations sur le patient, notamment son nom ou la référence de la prescription.

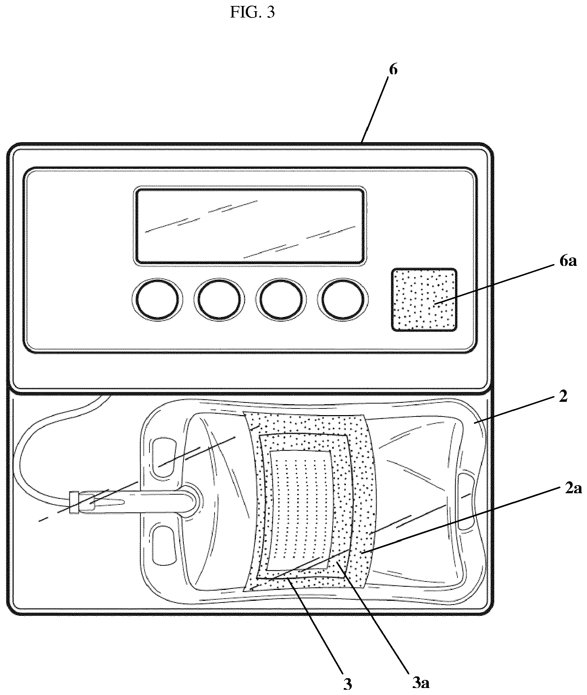

La FIG. 3 représente un mode de réalisation particulier dans lequel la pompe **6,** le réservoir **2** une fois positionné dans la pompe **6,** et l'étiquette détachable **3** une fois positionnée sur le réservoir **2** sont agencés de façon à permettre une comparaison visuelle des informations (IP) et (IE) présentes sur la pompe **6a** et sur l'étiquette **3a.** Ce mode de réalisation est notamment adapté lorsque les moyens d'identification sont des symboles ou un code couleur.

Dans ce mode de réalisation, les informations (IP) préprogrammées sont également reprises au moins en partie par des moyens d'identification visuels (IVP) **6a,** tel qu'un symbole ou un code couleur, auxquels les informations (IP) sont associées via l'établissement d'un code prédéterminé. Les informations reprises par les moyens d'identification visuels (IVP) **6a** sont par exemple la nature du principe actif et la concentration de celui-ci. Ces moyens d'identification visuels (IVP) **6a** sont apposés sur un emplacement de la pompe **6** prévu à cet effet par exemple sous la forme d'une étiquette. Dans ce mode de réalisation, les moyens d'identification de l'étiquette détachable **3** sont également des moyens d'identifications visuels (IVE) **3a** associés aux informations (IE) relatives à la solution médicamenteuse, telles qu'annoncées sur l'emballage, et répondant au même code que les moyens d'identification visuels (IVP) **6a** sur la pompe **6.** Ces moyens d'identification visuels (IVP) **6a** et (IVE) **3a,** respectivement sur la pompe **6** et sur l'étiquette **3,** sont agencés de façon à ce que, lorsque l'étiquette **3** est collée sur réservoir **2** et que le réservoir **2** est positionné dans la pompe **6,** lesdits moyens d'identification visuels sur la pompe (IVP) **6a** et sur l'étiquette (IVE) **3a** soient tous deux visibles et de préférence à proximité l'un de l'autre. Des moyens d'identification visuels (IVR) **2a** sont également présents sur le réservoir **2.** Les moyens d'indentifications visuels (IVR) **2a** sont associés à des informations (IR) relatives également à la solution médicamenteuse contenue dans le réservoir **2,** et répondent au même code que les moyens d'identification visuels (IVP) **6a** et (IVE) **3a.** Ils peuvent par exemple être disposés sur le réservoir **2** sur le pourtour de l'emplacement **4** adapté pour recevoir l'étiquette détachable **3** de façon à ce que lorsque l'étiquette 3 est positionnée sur cet emplacement **4,** les moyens d'identification visuels du réservoir (IVR) **2a** restent visibles et soient à proximité immédiate des moyens d'identification visuels (IVE) **3a** de l'étiquette **3.**

Dans ce mode de réalisation, la méthode sécurisée d'utilisation du kit de recharge d'une pompe à perfusion selon l'invention comprend les étapes suivantes :
- programmer une pompe à perfusion programmable **6** avec des informations (IP) relatives à la solution médicamenteuse à administrer par perfusion ;
- positionner des moyens d'identification visuels (IVP) **6a** sur la pompe à perfusion programmable **6,** lesdits moyens d'identification visuels (IVP) **6a** correspondant à des informations (IP) relatives à la solution médicamenteuse à administrer par perfusion ;
- détacher de l'emballage **1** l'étiquette détachable **3** comprenant des moyens d'identification visuels (IVE) **3a** et positionner ladite étiquette détachable **3** sur le réservoir **2** à l'emplacement prédéterminé **4** pour recevoir l'étiquette détachable **3** ; lesdits moyens d'identification visuels (IVE) **3a** correspondant à des informations (IE) relatives à la solution médicamenteuse contenue dans le réservoir **2** ;
- positionner le réservoir **2** dans la pompe à perfusion programmable **6** ;
- comparer les moyens d'identification visuels de l'étiquette (IVE) **3a** et de la pompe (IVP) **6a.**

Par exemple, lorsque les moyens d'identification visuels sont un code couleur, un code prédéterminé (échelle de couleur) associe une couleur donnée à une concentration de solution à perfuser donnée. Le praticien positionne, sur un emplacement de la pompe prévu à cet effet, une pastille de couleur (IVP) correspondant, selon le code prédéterminé, à la solution à perfuser telle que préprogrammée dans la pompe (IP). De manière alternative, le praticien positionne une pastille comprenant l'échelle de couleur ou la légende (IVP) définissant le code prédéterminé. De la même manière, l'étiquette détachable est au moins en partie colorée (par exemple sur le pourtour), selon le même code couleur prédéterminé, avec la couleur (IVE) correspondant à la solution à perfuser à laquelle elle est destinée (IE). Le pourtour de l'emplacement du réservoir adapté pour recevoir l'étiquette détachable peut également être coloré, toujours selon le même code prédéterminé, dans la couleur (IVR) correspondant à la solution à perfuser qu'il contient (IR). Lorsque l'utilisateur décolle l'étiquette de l'emballage principal pour la positionner sur l'emplacement prévu sur le réservoir, les couleurs de l'étiquette (IVE) et du réservoir (IVR) sont directement comparables permettant à l'utilisateur de repérer sans difficulté une erreur. De même, si les couleurs de l'étiquette (IVE) et du réservoir (IVR) correspondent, l'utilisateur positionne sans inquiétude le réservoir dans la pompe. La couleur (ou l'échelle de couleur) de la pompe (IVP) d'une part et les couleurs de l'étiquette et du réservoir (IVE et IVR) d'autre part, sont alors directement comparables et l'utilisateur peut vérifier sans difficulté la concordance entre les caractéristiques de la solution à perfuser préprogrammée dans la pompe (IP) et les caractéristiques de la solution à perfusée contenue dans le réservoir (IE et IR). De tels moyens d'indentification visuels permettent ainsi une comparaison simple et directe par l'utilisateur des informations (IP) préprogrammées et des informations (IE et IR) relatives à la solution médicamenteuse à perfuser. De plus, du fait de cette simplicité, l'utilisateur, même non averti, vérifie pratiquement inévitablement et de manière systématique la correspondance des informations (IP) préprogrammées et des informations (IE et IR) relatives à la solution médicamenteuse à perfuser. Ainsi, cette vérification fait office de détrompeur. La méthode selon l'invention permet ainsi à l'utilisateur de se rendre compte d'une erreur avant la mise en marche de la pompe à perfusion.

Ce mode de réalisation, tout en permettant de diminuer les risques d'erreur liés à la recharge d'une pompe à perfusion, a comme avantage d'être peu onéreux comparé à des méthodes de sécurisation utilisant des pompes à perfusion comprenant des dispositifs de sécurisation complexes. Elle peut également être utilisée en complément des dispositifs de sécurisation existants.

Dans un autre mode de réalisation, la pompe à perfusion comprend des moyens de détection et des moyens de contrôle. Les moyens de détections doivent permettre la détection des informations (IE) contenues dans les moyens d'identifications de l'étiquette détachable. Les moyens de détection peuvent être par exemple un capteur de couleur, un lecteur de code barre, un lecteur de code en deux dimensions ou un lecteur des moyens de stockage de données, notamment de puce RFID. Les moyens de contrôle permettent la comparaison des informations détectées (IE) et des informations préprogrammées (IP).

Les moyens de contrôle permettent une comparaison automatique des informations (IP) et (IE).

Les moyens de contrôle peuvent être reliés à des moyens d'avertissement, tels qu'un signal sonore et/ou visuel. Lorsque les moyens de contrôle détectent une discordance entre les informations détectées (IE) et des informations préprogrammées (IP), les moyens de contrôle activent les moyens d'avertissement de façon à avertir l'utilisateur de l'erreur.

Les moyens de contrôle peuvent également être reliés à des moyens de blocage. Lorsque les moyens de contrôle détectent une discordance entre les informations détectées (IE) et des informations préprogrammées (IP), les moyens de contrôle activent les moyens de blocage de façon à empêcher la délivrance de la solution médicamenteuse à perfuser.

En particulier, les moyens d'avertissement et/ou les moyens de blocages sont activés lorsque l'étiquette détachable n'est pas positionnée à l'emplacement adapté pour la recevoir. En effet, dans ce cas aucune information (IE) ne pouvant être détectée il ne peut y avoir correspondance des informations détectées (IE) avec les informations préprogrammées (IP).

Dans ce mode de réalisation, la présence de l'étiquette contenant les moyens d'identification sur le réservoir fournit un niveau supplémentaire de sécurité en permettant une comparaison automatique des informations (IE) contenues dans ces moyens d'identification avec les informations préprogrammées (IP) de la pompe à perfusion à laquelle le kit de recharge est destiné. Cela permet de prévenir les incidents dus par exemple à la fourniture d'une solution médicamenteuse à perfuser à une concentration autre que celle prévue par la préprogrammation de la pompe. En particulier, lorsque les moyens d'identification sont des supports de stockage de données, il est possible de prévoir une double entrée des conditions d'administration de la solution médicamenteuse, à savoir, une première fois par le personnel médical qui préprogramme la pompe et renseigne les informations préprogrammées (IP) ; et une seconde fois par le personnel médical qui délivre le kit de recharge de la pompe et renseigne les informations (IE) contenues dans le support de stockage. Ces informations sont détectées par les moyens de détection et comparées par les moyens de contrôle qui peuvent éventuellement activer les moyens d'avertissement et/ou les moyens de blocage lorsque les conditions d'administration entrées dans les informations préprogrammées (IP) et celles entrées dans les informations (IE) contenues dans les moyens de stockage ne correspondent pas. Ce mode de réalisation fournit donc également un niveau de sécurité supplémentaire par rapport aux éventuelles erreurs de programmation de la pompe.

L'étiquette détachable doit être positionnée sur le réservoir et les informations relatives au nom du patient et au numéro de prescription doivent être inscrites sur cette étiquette. Le risque d'oublier de coller l'étiquette sur le réservoir existe ce qui peut être préjudiciable à la sécurité du patient et à la traçabilité des analgésiques de niveau III. Cependant, dans ce mode de réalisation, les moyens de détection et de contrôle permettent de détecter cet oubli et d'avertir l'utilisateur, voire d'empêcher la mise en place de la perfusion. Le risque d'oublier de coller l'étiquette sur le réservoir est donc considérablement réduit. De plus, lorsque l'utilisateur colle l'étiquette sur le réservoir, il voit nécessairement si les mentions concernant le nom du patient et la référence de la prescription sont inscrites et est donc incité à les renseigner si elles sont absentes. Ainsi, l'utilisateur sera moins enclin à oublier de reporter les informations lors de la mise en place du système de perfusion. En particulier, lorsque les moyens d'identification sont des moyens de stockage, les informations relatives au nom du patient et au numéro de prescription peuvent y être ajoutées par exemple par le personnel médical délivrant le kit de recharge, et les moyens de contrôle peuvent être prévus de façon à activer les moyens d'avertissement et/ou les moyens de blocage si ces informations ne sont pas détectées par les moyens de détection. Ainsi, dans le cas des analgésiques de classe III notamment, la méthode de la présente invention permet de réduire les risques d'erreur et d'améliorer la traçabilité de ces substances.

La présente invention porte également sur un dispositif sécurisé de délivrance d'une solution médicamenteuse par perfusion comprenant :
- un réservoir contenant une solution médicamenteuse à administrer par perfusion ; et
- une pompe à perfusion préprogrammable ;
caractérisé en ce que le réservoir comprend des moyens d'identification contenant des informations (IR) sur la solution médicamenteuse à administrer par perfusion ; et
la pompe à perfusion comprend des moyens de détection permettant de détecter les informations (IR) contenues dans les moyens d'identification et des moyens de contrôle permettant de comparer les informations détectées (IR) avec des informations préprogrammées (IP).

Le réservoir, les moyens d'identification, la pompe à perfusion programmable, les moyens de détection et les moyens de contrôle sont tels que définis ci-dessus. Notamment, la pompe peut comprendre en outre des moyens d'avertissement et/ou des moyens de blocage, tels que définis précédemment, reliés aux moyens de contrôle.

Les moyens d'identification peuvent être sur le réservoir lui-même. De préférence, ils sont apposés sur le réservoir par une étiquette détachable telle que définie ci-dessus. Dans ce cas, les informations (IR) sont des informations (IE) contenues dans les moyens d'identification de l'étiquette.

Le dispositif selon l'invention comprend également une tubulure et un cathéter permettant l'administration au patient de la solution médicamenteuse à perfuser. La tubulure est destinée à relier le réservoir au cathéter. Lors de l'utilisation, elle est positionnée dans la pompe à perfusion de façon à ce que la pompe puisse contrôler le débit de solution médicamenteuse s'écoulant dans la tubulure et à administrer au patient.

Un tel dispositif permet la mise en oeuvre de la méthode sécurisée d'utilisation du kit de recharge selon l'invention.

Un autre objet de la présente invention porte sur une méthode sécurisée de préparation du kit de recharge d'une pompe à perfusion tel que défini précédemment comprenant les étapes suivantes :
- fournir à l'emballage principal des moyens d'identification contenant des informations (I);
- fournir à chacun des autres composants (n) du kit des moyens d'identification contenant des informations (iₙ);
- positionner chaque composant (n) du kit dans l'emballage principal ;
- détecter les informations (I) contenues dans les moyens d'identification de l'emballage principal d'une part et les informations (iₙ) de chaque composant (n) du kit d'autre part ; et
- vérifier la présence des composants (n) et la concordance des informations (I) de l'emballage principal avec les informations (iₙ) de chacun des autres composants (n) du kit.

Chaque composant (n) du kit comprend des moyens d'identification contenant des informations (iₙ). Les moyens d'identification fournis à l'emballage principal du kit contiennent des informations (I). Les informations (I) sont relatives au moins au réservoir contenant la solution médicamenteuse à perfuser, de préférence les informations (I) sont relatives à chacun des composants (n) destiné à être contenu dans l'emballage principal. Les moyens d'identification fournis aux autres composants (n) du kit, dont notamment le réservoir contenant la solution médicamenteuse, contiennent des informations (iₙ). Dans une première alternative, ces informations (iₙ) sont relatives au réservoir contenant la solution médicamenteuse à perfuser auquel le composant (n) doit être associé. Dans ce cas les informations (I) sont également limitées à des informations relatives au réservoir contenant la solution médicamenteuse à perfuser que l'emballage principal doit contenir. Dans une seconde alternative, les autres composants comprennent des moyens d'identifications contenant des informations (iₙ) relatives audit composant. Dans ce cas les informations (I) sont les informations relatives à chacun des composants (n) que l'emballage principal doit contenir.

Par « autres composants du kit » il est fait référence, outre au réservoir contenant la solution médicamenteuse à perfuser, d'autres composants tels qu'une tubulure ou une notice. A chacun de ces composants sont associées des informations (iₙ) et des moyens d'identification contenant ces informations.

Les moyens d'identification sont tels que définis ci-dessus et peuvent être par exemple un code couleur, un code barre, un code en deux dimensions ou une puce RFID.

Les informations contenues dans les moyens d'identifications sont détectées par des moyens de détection adaptés aux moyens d'identification choisis. Les moyens de détection peuvent être un système de reconnaissance de couleur par analyse d'image, un lecteur de code barre, un lecteur de code à deux dimensions ou un lecteur de puce RIFD.

Dans le cas d'un code couleur, une couleur peut par exemple être associée à une solution médicamenteuse à perfuser donnée, c'est-à-dire contenant un principe actif particulier à une concentration donnée, et éventuellement à une capacité de réservoir donnée. La couleur est alors apposée sur au moins une partie du réservoir lui-même. Les parois du réservoir devant rester transparentes, la couleur est apposée par exemple en colorant une partie seulement du réservoir (par exemple le pourtour d'une poche souple, le pourtour de l'emplacement adapté pour recevoir l'étiquette détachable, le raccord ou la valve jointe à l'orifice du réservoir), en y collant une étiquette colorée ou en colorant un emballage primaire renfermant le réservoir. Cette couleur peut être apposée sur chacun des composants du kit, y compris sur l'emballage principal. La couleur peut être détectée par simple inspection visuelle ou par des moyens de détection automatiques, notamment par reconnaissance de couleur par analyse d'image. De préférence, la zone colorée sur chaque composant du kit est suffisamment étendue pour permettre une détection de la couleur par les moyens de détection. Ainsi, il est possible de procéder efficacement à la vérification de la conformité de la préparation du kit de recharge en vérifiant que le code couleur est identique sur l'emballage principal et sur tous les composants du kit. Selon un mode de réalisation avantageux, l'emballage principal comprend une fenêtre et la zone colorée de chacun des composants du kit est disposée de telle sorte qu'elle apparaisse par cette fenêtre. Ainsi, par simple inspection visuelle, il sera possible de détecter si le kit est conforme ou pas, une seule couleur devant apparaître par cette fenêtre.

De préférence, les moyens d'identification sont une puce RFID. Dans ce cas, une puce RFID contenant les informations (I) est fournie à l'emballage principal et une puce RFID contenant des informations (iₙ) est fournie à chaque composant (n) du kit autre que l'emballage principal. Les informations (iₙ) comprennent des informations relatives au composant (n) auquel elles sont associées. Par exemple, la puce RFID du réservoir contenant la solution médicamenteuse à perfuser peut contenir des informations relatives à la nature du principe actif, à la concentration de celui-ci, à la capacité du réservoir ou encore au numéro de lot ou à la date limite d'utilisation. Les informations (I) comprennent des informations relatives à chacun des composants (n) que l'emballage principal doit contenir, notamment le nombre de composants et la nature de chacun de ces composants. En particulier, les informations (I) peuvent comprendre chacune des informations (iₙ). Un lecteur de puce RFID permet de détecter chacune des puces RFID et de lire les informations qu'elles comprennent. Le lecteur RFID détecte chacune des puces RFID du kit, ce qui permet de vérifier la présence de chacun des composants (n). Des moyens de contrôle permettent de vérifier la correspondance de chacune des informations (iₙ) détectées pour chacun des composants (n) avec les informations relatives à ce composant contenues dans les informations (I) de l'emballage principal. Cette méthode permet par conséquent de sécuriser la préparation d'un kit de recharge de pompe à perfusion en contrôlant d'une part la présence de chacun des composants du kit et d'autre part la conformité du contenu du kit avec les informations contenues sur l'emballage. De plus, les puces RFID pouvant être lues à distance par des moyens de détection peu coûteux, ces vérifications peuvent être faites non seulement au cours de la chaîne de préparation des kits mais également à tout moment tout au long de la chaîne logistique jusqu'à l'utilisateur. Cette méthode permet ainsi d'éviter les erreurs de préparation notamment concernant la fourniture dans le kit d'un réservoir contenant une solution médicamenteuse à perfuser ayant une concentration non conforme avec les informations de l'emballage du kit, mais également d'améliorer la traçabilité pour les analgésiques de type III.

Dans le cas où les moyens d'identification sont des symboles du type codes barre ou des codes à deux dimensions, un symbole contenant les informations (I) est fourni à l'emballage principal et un symbole contenant des informations (iₙ) est fourni à chaque composant (n) du kit autre que l'emballage principal. Les informations (I) et (iₙ) sont telles que définies ci-dessus dans le cas de puces RFID. Dans ce mode de réalisation, les symboles de chaque composant (n) du kit et de l'emballage principal doivent être scannés individuellement par un lecteur du type lecteur de codes barre ou lecteur de codes à deux dimensions pour que les informations (iₙ) et (I) puissent être détectées. Selon un mode de réalisation avantageux, l'emballage principal comprend une fenêtre et le symbole de chacun des composants du kit est disposé de telle sorte qu'il apparaisse par cette fenêtre. Ainsi le procédé de l'invention peut être effectué une fois les opérations d'emballage terminées pour vérifier la conformité du kit.

## Revendications

1. Kit de recharge d'une pompe à perfusion comprenant :
- un emballage (1) auquel est attaché une étiquette détachable (3); et
- un réservoir (2) qui est une poche souple prête à l'emploi contenant une solution médicamenteuse à administrer par perfusion ;
ladite étiquette détachable (3) comprend des moyens d'identification visuels (IVE) (3a) autres qu'un message écrit qui sont des symboles, un code couleur ou une combinaison de ceux-ci, **caractérisé en ce qu'**un emplacement (4) est prévu à la surface du réservoir (2), ledit emplacement (4) étant adapté pour recevoir l'étiquette détachable (3),
ledit réservoir (2) comprend des moyens d'identification visuels (IVR) (2a) autres qu'un message écrit qui sont des symboles, un code couleur ou une combinaison de ceux-ci, et
les moyens d'identification visuels du réservoir (IVR) (2a) répondant au même code prédéterminé, symbole prédéterminé ou combinaison de ceux-ci, que les moyens d'identification visuels de l'étiquette (IVE) (3a).

2. Kit selon la revendication 1, **caractérisé en ce que** la solution médicamenteuse à administrer par perfusion comprend un analgésique de type III, de préférence de la morphine, de l'oxicodone ou du fentanyl.

3. Kit selon la revendication 1 ou 2, **caractérisé en ce que** l'emplacement (4) adapté pour recevoir l'étiquette détachable (3) comprend une alerte visuelle (4a).

4. Kit selon l'une quelconque des revendications 1 à 3, **caractérisé en ce que** les moyens d'identification visuels (IVE) (3a) correspondent à des informations (IE) relatives à la solution médicamenteuse à perfuser annoncées sur l'emballage (1) et les moyens d'identification (IVR) (2a) correspondent à des informations (IR) relatives à la solution médicamenteuse à perfuser contenue dans le réservoir (2).

5. Kit selon l'une quelconque des revendications 1 à 4, **caractérisé en ce que** les moyens d'identification visuels (IVR) (2a) sont disposés au voisinage de l'emplacement (4) prédéterminé pour recevoir l'étiquette détachable (3) de façon à ce que lesdits moyens d'identification visuels (IVR) (2a) restent visibles au moins en partie lorsque l'étiquette (3) est positionnée sur l'emplacement (4).

6. Méthode sécurisée d'utilisation du kit de recharge d'une pompe à perfusion tel que défini dans les revendications 1 à 5 comprenant les étapes suivantes :
- programmer une pompe à perfusion programmable (6) avec des informations (IP) relatives à la solution médicamenteuse à administrer par perfusion ;
- positionner des moyens d'identification visuels (IVP) (6a) sur la pompe à perfusion programmable (6), lesdits moyens d'identification visuels (IVP) (6a) correspondant à des informations (IP) relatives à la solution médicamenteuse à administrer par perfusion ;
- détacher de l'emballage (1) l'étiquette détachable (3) comprenant des moyens d'identification visuels (IVE) (3a) et positionner ladite étiquette détachable (3) sur le réservoir (2) à l'emplacement prédéterminé (4) pour recevoir l'étiquette détachable (3); lesdits moyens d'identification (IVE) (3a) contenant des informations (IE) relatives à la solution médicamenteuse contenue dans le réservoir (2);
- positionner le réservoir (2) dans la pompe à perfusion programmable (6); et
- comparer les moyens d'identification visuels de l'étiquette (IVE) (3a) et de la pompe (IVP) (6a).

7. Méthode selon la revendication 6, **caractérisée en ce que** les moyens d'identification visuels (IVE) (3a) et (IVP) (6a) sont des symboles, un code couleur ou une combinaison de ceux-ci.

8. Méthode selon la revendication 6 ou 7, **caractérisée en ce que** les moyens d'identification visuels (IVP) (6a) et (IVE) (3a) sont agencés de façon à ce que, lorsque l'étiquette (3) est collée sur le réservoir (2) et que le réservoir (2) est positionné dans la pompe (6), lesdits moyens d'identification visuels sur la pompe (IVP) (6a) et sur l'étiquette (IVE) (3a) soient tous deux visibles et de préférence à proximité l'un de l'autre.

## Patentansprüche

1. Wiederbefüllset für eine Infusionspumpe, mit:
- einer Verpackung (1), an der ein entfernbares Etikett (3) angebracht ist; und
- einem Behältnis (2), das ein flexibler Beutel ist, der dafür vorgesehen ist, eine Arzneimittellösung für eine Verabreichung durch Infusion zu enthalten;
wobei das ablösbare Etikett (3) andere visuelle Identifikationsmittel (IVE) (3a) als eine geschriebene Nachricht aufweist, die Symbole, ein Farbcode oder eine Kombination daraus sind,
**dadurch gekennzeichnet, dass** eine Stelle (4) an der Fläche des Behältnisses vorgesehen ist, wobei die Stelle angepasst ist, ein entfernbares Etikett (3) zu empfangen,
wobei das Behältnis (2) andere visuelle Identifikationsmittel (IVR) (2a) als eine geschriebene Nachricht aufweist, die Symbole, ein Farbcode oder eine Kombination daraus sind, und
wobei die visuellen Identifikationsmittel des Behältnisses (IVR) (2a) demselben vorbestimmten Code, Symbol oder Kombination daraus entspricht wie die visuellen Identifikationsmittel des Etiketts (IVE) (3a).

2. Set nach Anspruch 1, **dadurch gekennzeichnet, dass** die durch Infusion zu verabreichende Arzneimittellösung ein Typ III Analgetikum, vorzugsweise Morphin, Oxycodon oder Fentanyl, aufweist.

3. Set nach Anspruch 1 oder 2, **dadurch gekennzeichnet, dass** die zum Empfangen des entfernbaren Etiketts (3) angepasste Stelle eine visuelle Warnung (4a) aufweist.

4. Set nach einem der Ansprüche 1 bis 3, **dadurch gekennzeichnet, dass** die visuellen Identifikationsmittel (IVE) (3a) mit Informationen (IE) bezüglich der durch Infusion zu verabreichenden Arzneimittellösung korrespondieren, die auf der Verpackung (1) angegeben sind, und die visuellen Identifikationsmittel (IVR) (2a) mit Informationen (IR) bezüglich der durch Infusion zu verabreichenden Arzneimittellösung korrespondieren, die in dem Behältnis (2) enthalten sind.

5. Set nach einem der Ansprüche 1 bis 4, **dadurch gekennzeichnet, dass** die visuellen Identifikationsmittel (IVR) (2a) so benachbart zu der Stelle (4) angeordnet ist, die zum Empfangen des entfernbaren Etiketts (3) vorgesehen ist, dass die visuellen Identifikationsmittel (IVR) (2a) zumindest teilweise sichtbar bleiben, wenn das Etikett (3) auf der Stelle (4) positioniert ist.

6. Verfahren für eine sichere Verwendung des Wiederbefüllsets für eine Infusionspumpe, wie in den Ansprüchen 1 bis 5 definiert, dass die folgenden Schritte umfasst:
- Programmieren einer programmierbaren Infusionspumpe (6) mit den Informationen (IP) bezüglich der durch Infusion zu verabreichenden Arzneimittellösung;
- Positionieren der visuellen Identifikationsmittel (IVP) (6a) auf der programmierbaren Infusionspumpe (6), wobei die visuellen Identifikationsmittel (IVP) (6a) mit Informationen (IP) bezüglich der durch Infusion zu verabreichenden Arzneimittellösung korrespondieren;
- Entfernen des entfernbaren Etiketts (3), das die visuellen Identifikationsmittel (IVE) (3a) aufweist, von der Verpackung (1) und Positionieren des entfernbaren Etiketts (3) auf dem Behältnis (2) an der zum Empfang des entfernbaren Etiketts (3) vorbestimmten Stelle (4), wobei die Identifikationsmittel (IVE) (3a) Informationen (IE) bezüglich der in dem Behältnis (2) enthaltenden Arzneimittellösung aufweisen;
- Positionieren des Behältnisses (2) in der programmierbaren Infusionspumpe (6); und
- Vergleichen der visuellen Identifikationsmittel des Etiketts (IVE) (3a) und der Pumpe (IVP) (6a).

7. Verfahren nach Anspruch 6, **dadurch gekennzeichnet, dass** die visuellen Identifikationsmittel (IVE) (3a) und (IVP) (6a) Symbole, ein Farbcode oder eine Kombination daraus sind.

8. Verfahren nach Anspruch 6 oder 7, **dadurch gekennzeichnet, dass** die visuellen Identifikationsmittel (IVP) (6a) und (IVE) (3a) so angeordnet sind, dass die visuellen Identifikationsmittel auf der Pumpe (IVP) (6a) und auf dem Etikett (IVE) (3a) beide sichtbar und vorzugsweise nah beieinander sind, wenn das Etiketts (3) auf das Behältnis (2) geklebt ist und das Behältnis (2) in der Pumpe (6) positioniert ist.

## Claims

1. Kit for recharging an infusion pump, comprising:
- a packaging (1) to which a detachable label (3) is attached; and
- a reservoir (2) which is a ready-to-use flexible bag containing a medicamentous solution to be administered by infusion;
said detachable label (3) comprises visual identification means (IVE) (3a) other than a text message, which are symbols, a colour code or a combination thereof, **characterised in that** a designated space (4) is provided on the surface of the reservoir (2), said designated space (4) being designed to receive the detachable label (3), said reservoir (2) comprises visual identification means (IVR) (2a) other than a text message, which are symbols, a colour code or a combination thereof, and
the visual identification means of the reservoir (IVR) (2a) corresponds to the same predetermined code, predetermined symbol or combination thereof as the visual identification means of the label (IVE) (3a).

2. Kit as claimed in claim 1, **characterised in that** the medicamentous solution to be administered by infusion comprises a type III analgesic, preferably morphine, oxycodone or fentanyl.

3. Kit as claimed in claim 1 or 2, **characterised in that** the designated space (4) designed to receive the detachable label (3) has a visual warning (4a).

4. Kit as claimed in any one of claims 1 to 3, **characterised in that** the visual identification means (IVE) (3a) correspond to information (IE) relating to the medicamentous solution to be infused specified on the packaging (1) and the visual identification means (IVR) (2a) correspond to information (IR) relating to the medicamentous solution to be infused contained in the reservoir (2).

5. Kit as claimed in any one of claims 1 to 4, **characterised in that** the visual identification means (IVR) (2a) are disposed in the vicinity of the predetermined designated space (4) for receiving the detachable label (3) so that said visual identification means (IVR) (2a) remain at least partially visible when the label (3) is positioned on the designated space (4).

6. Security-protected method of using a kit for recharging an infusion pump as defined in claims 1 to 5, comprising the following steps:
- programming a programmable infusion pump (6) with information (IP) relating to the medicamentous solution to be administered by infusion;
- positioning visual identification means (IVP) (6a) on the programmable infusion pump (6), said visual identification means (IVP) (6a) corresponding to information (IP) relating to the medicamentous solution to be administered by infusion;
- detaching the detachable label (3) containing the visual identification means (IVE) (3 a) from the packaging (1) and positioning said detachable label (3) on the reservoir (2) in the predetermined designated space (4) for receiving the detachable label (3); said identification means (IVE) (3a) containing information (IE) relating to the medicamentous solution contained in the reservoir (2);
- positioning the reservoir (2) in the programmable infusion pump (6); and
- comparing the visual identification means of the label (IVE) (3a) and the pump (IVP) (6a).

7. Method as claimed in claim 6, **characterised in that** the visual identification means (IVE) (3a) and (IVP) (6a) are symbols, a colour code or a combination thereof.

8. Method as claimed in claim 6 or 7, **characterised in that** the visual identification means (IVP) (6a) and (IVE) (3a) are disposed such that when the label (3) is adhered to the reservoir (2) and the reservoir (2) is positioned in the pump (6), said visual identification means on the pump (IVP) (6a) and on the label (IVE) (3a) are both visible and preferably close to one another.
